# EUROPEAN PATENT SPECIFICATION

(11) **EP 4 235 170 B1**
(45) Date of publication and mention of the grant of the patent: **25.06.2025**
(21) Application number: 23154297.8
(22) Date of filing: 31.01.2023
(51) Int. Cl.: G01N 30/06, G01N 30/74

(54) **METHOD OF QUANTIFICATION OF FREE SULFUR**
VERFAHREN ZUR QUANTIFIZIERUNG VON FREIEM SCHWEFEL
PROCÉDÉ DE QUANTIFICATION DE SOUFRE LIBRE

(30) Priority: 25.02.2022 JP 2022028078
(43) Date of publication of application: 30.08.2023
(73) Proprietor: Sumitomo Rubber Industries, Ltd., Kobe-shi, Hyogo 651-0072 (JP)
(72) Inventor: IWASAKI, Yuko, Kobe-shi, Hyogo, 651-0072 (JP); SAKAGUCHI, Yumi, Kobe-shi, Hyogo, 651-0072 (JP); YAMADA, Hiroaki, Kobe-shi, Hyogo, 651-0072 (JP)
(74) Representative: Manitz Finsterwald Patent- und Rechtsanwaltspartnerschaft mbB

(56) References cited:
- CN-A- 108 752 670
- JP-A- 2020 528 843
- JP-B2- 6 665 629
- US-A1- 2018 305 504

## Description

### TECHNICAL FIELD

The present invention relates to a method of quantification of free sulfur.

### BACKGROUND ART

Rubber contains unreacted sulfur, i.e., free sulfur which does not contribute to the crosslinking reaction of the rubber. For example, Non-Patent Literature 1 discloses a method for quantification of free sulfur including analyzing the extract obtained by immersion in THF. Patent Literature 1 discloses a free sulfur test wherein a rubber-plastic blend is cut into small particles, sulfur is extracted by using toluene as an extractant, and the sulfur content is measured by a liquid chromatograph. Patent Literature 2 discloses a measurement of free sulfur content wherein a rubber vulcanizate is subjected to immersion extraction with tetrahydrofuran (THF), and the sulfur content of the liquid is determined by high-performance liquid chromatography (HPLC). Patent Literature 3 discloses a method of quantifying components of a rubber composition which involves obtaining a chromatogram by analyzing a solvent extract of a rubber composition using gas chromatography. However, the free sulfur content data obtained by conventional methods may vary and therefore it is desirable to provide a highly accurate quantitative method.

### CITATION LIST

### NON PATENT LITERATURE

Non-Patent Literature 1: "Quantitative Analysis of Free Sulfur Content in the Vulcanized Rubber by HPLC", Journal of The Society of Rubber Industry, Japan, Vol. 81, No. 2 (2008)
Patent Literature 1: CN 108 752 670 A
Patent Literature 2: US 2018/305504 A1
Patent Literature 3: JP 6665629 B2

### SUMMARY OF INVENTION

### TECHNICAL PROBLEM

The present invention aims to solve the aforementioned problem and provide a method of quantification of free sulfur that can accurately measure the free sulfur content of a rubber composition.

### SOLUTION TO PROBLEM

The present invention relates to a method of quantification of free sulfur in a rubber composition, the method including extracting the free sulfur in the rubber composition with toluene, wherein the quantification of free sulfur is performed by chromatography, and the free sulfur is detected using an ultraviolet-visible detector at a measurement wavelength of 280 to 330 nm.

### ADVANTAGEOUS EFFECTS OF INVENTION

The present invention relates to a method of quantification of free sulfur in a rubber composition, which includes extracting the free sulfur in the rubber composition with toluene, wherein the quantification of free sulfur is performed by chromatography, and the free sulfur is detected using an ultraviolet-visible detector at a measurement wavelength of 280 to 330 nm. Such a method can accurately measure the free sulfur content of the rubber composition.

### BRIEF DESCRIPTION OF DRAWINGS

FIG. 1 is an example of a chromatogram obtained by subjecting a toluene extract of a rubber composition to HPLC with a UV-VIS detector (measurement wavelength: 264 nm).
FIG. 2 is an example of a chromatogram obtained by subjecting a toluene extract of a rubber composition to HPLC with a UV-VIS detector (measurement wavelength: 300 nm).
Fig. 3 is an example of a diagram illustrating the free sulfur contents analyzed by the quantitative methods of a comparative example and an example.

### DESCRIPTION OF EMBODIMENTS

The present invention relates to a method of quantification of free sulfur in a rubber composition. The method includes extracting the free sulfur in the rubber composition with toluene.

The present inventors first found the following problem: when an extract obtained by immersing a rubber composition in tetrahydrofuran (THF) is separated by gel permeation chromatography (GPC) and detected with an ultraviolet-visible detector at a measurement wavelength of 264 nm, and the amount of free sulfur is quantified from the peak intensity or area of the chromatogram, the measured free sulfur content may vary, particularly if the rubber composition (sample) contains insoluble sulfur.

Then, the present inventors investigated the cause of this problem and assumed that a transition from insoluble sulfur to soluble sulfur may occur in a sample immersed in THF, thereby increasing the determined (measured) free sulfur content.

In contrast, the method of the present invention uses toluene to extract free sulfur, and a transition from insoluble sulfur to soluble sulfur is prevented in the toluene extraction. Thus, the method can accurately measure free sulfur.

Here, the toluene extraction may have concerns that a UV peak attributed to toluene cannot be separated from a UV peak attributed to free sulfur. However, for example, when the UV detection wavelength is changed from 264 nm to 280 to 330 nm, the detection of the UV peak of toluene is reduced, enabling more accurate quantification of free sulfur.

The method of quantification of free sulfur of the present invention includes extracting free sulfur in a rubber composition (a rubber composition containing sulfur) with toluene.

In the present invention, the term "free sulfur" means the sulfur that is present in a rubber composition (sample) and does not contribute to a crosslinking reaction.

The rubber composition (sample) to be subjected to the present invention contains at least a rubber component and sulfur.

Non-limiting examples of usable rubber components include diene rubbers. Examples of the diene rubbers include isoprene-based rubbers, polybutadiene rubbers (BR), styrene-butadiene rubbers (SBR), styrene-isoprene-butadiene rubbers (SIBR), ethylene-propylene-diene rubbers (EPDM), chloroprene rubbers (CR), and acrylonitrile-butadiene rubbers (NBR). Other examples include butyl-based rubbers and fluororubbers. These may be used alone or in combinations of two or more.

Any sulfur may be used, including materials commonly used as cross-linking agents in the rubber industry. Examples include powdered sulfur, precipitated sulfur, colloidal sulfur, insoluble sulfur, highly dispersible sulfur, and soluble sulfur. In particular, the present invention can also be suitably applied to a rubber composition (sample) containing insoluble sulfur that has concerns about transition from insoluble sulfur to soluble sulfur. Usable commercial products of sulfur are available from Tsurumi Chemical Industry Co., Ltd., Karuizawa Sulfur Co., Ltd., Shikoku Chemicals Corporation, Flexsys, Nippon Kanryu Industry Co., Ltd., Hosoi Chemical Industry Co., Ltd., etc. These may be used alone or in combinations of two or more.

Although the rubber composition (sample) may have any sulfur content, the sulfur content per 100 parts by mass of the rubber component content is preferably 0.5 parts by mass or more, more preferably 1.5 parts by mass or more, still more preferably 2.0 parts by mass or more, while it is preferably 3.5 parts by mass or less, more preferably 3.0 parts by mass or less, still more preferably 2.8 parts by mass or less. With the sulfur content within the range indicated above, the free sulfur tends to be accurately measured.

The rubber composition (sample) may contain additional components.

Non-limiting examples of the additional components include materials known in the rubber field, including, for example, fillers (e.g., inorganic fillers such as silica, carbon black, calcium carbonate, talc, alumina, clay, aluminum hydroxide, aluminum oxide, and mica), silane coupling agents, plasticizers (e.g., oils, liquid resins, and solid resins), antioxidants, waxes, stearic acid, zinc oxide, and vulcanization accelerators. These may be used alone or in combinations of two or more.

The quantitative method of the present invention is applicable to both unvulcanized and vulcanized rubber compositions. The method of the present invention can prevent transition from insoluble sulfur to soluble sulfur by using toluene instead of THF. Thus, the method can be suitably applied to unvulcanized rubber compositions, particularly unvulcanized rubber compositions containing insoluble sulfur, to accurately measure the free sulfur content of the samples.

The rubber composition (sample) may be prepared as an unvulcanized rubber composition, for example, by kneading the above-mentioned components using a rubber kneading machine such as an open roll mill or a Banbury mixer. Moreover, the unvulcanized rubber composition may then be vulcanized to prepare a vulcanized rubber composition.

In the present invention, toluene is used to perform the step of extracting the free sulfur in the rubber composition (sample). From the standpoint of increasing the extraction efficiency, the rubber composition (sample) to be subjected is desirably cut into fine pieces and weighed precisely. The size of each cut sample piece is preferably 1 to 50 mm³, more preferably 3 to 30 mm³.

In the above-described step, various known extraction methods can be used, such as immersion extraction and Soxhlet extraction.

The immersion extraction may be carried out by a known technique, such as immersing the rubber composition (sample) in toluene at 15 to 60°C. The immersion time may be, for example, 2 to 72 hours, but is not limited thereto. During the immersion, stirring with a stirrer may be performed as needed.

The Soxhlet extraction may be carried out by a known technique. For example, preferably, heating is performed at a temperature of not lower than the boiling point of toluene because once heated toluene vaporizes, it liquefies again to act as an extraction solvent. The extraction time may be, for example, 2 to 72 hours, but is not limited thereto.

The extraction by the step provides an extract containing free sulfur.

The extract can be directly used as an analysis sample and subjected to an instrumental analyzer. Alternatively, the extract may be diluted as appropriate with an organic solvent to adjust the free sulfur concentration and then used as an analysis sample.

Chromatography is used to separate the free sulfur from the multiple components in the extract.

Examples of the chromatography include liquid chromatography and gas chromatography, with liquid chromatography being desirable. The liquid chromatography is preferably high performance liquid chromatography (HPLC). Gel permeation chromatography (GPC) is also desirable.

Examples of the liquid chromatography include normal phase chromatography, reverse phase chromatography, and gel permeation chromatography.

The free sulfur thus separated from the multiple components in the extract can be detected by various detection devices. Examples of usable detection devices include commonly and widely used detectors such as ultraviolet-visible detectors (UV-VIS detectors), photodiode array detectors (PDA detectors), differential refractive index detectors (RI detectors), fluorescence detectors (FL detectors), and evaporative light scattering detectors (ELSD detectors). Among these, UV-VIS detectors or PDA detectors are desirable from the standpoint of accurate measurement.

The quantification can be performed as follows: the extract is subjected to chromatography and detected with a detection device to output peaks with the horizontal axis representing the retention time in the column and the vertical axis representing the peak intensity; and among the peaks, the intensity or area of the peak attributed to free sulfur is read. Here, the free sulfur content can be determined from the peak intensity or area of an unknown analysis sample by subjecting solutions of various concentrations (standard sample solutions) to an instrumental analyzer to create a calibration curve.

From the standpoint of accurate measurement, the quantitative method of the present invention includes detecting the free sulfur using a UV-VIS detector at a measurement wavelength of 280 to 330 nm.

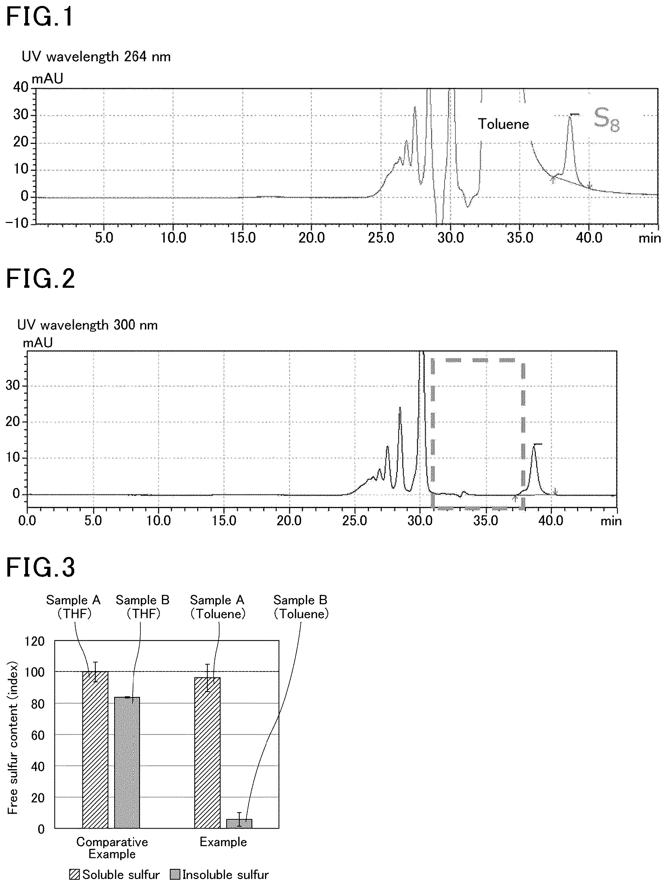

FIGs. 1 and 2 are each a chromatogram obtained by subjecting a toluene extract of a rubber composition (sample) to HPLC with a UV-VIS detector or a PDA detector. FIG. 1 shows the result at a measurement wavelength of 264 nm, and FIG. 2 shows the result at a measurement wavelength of 300 nm.

In FIG. 1 at a measurement wavelength of 264 nm, a peak attributed to toluene is also detected and overlaps with a peak attributed to free sulfur, while in FIG. 2 at a measurement wavelength of 300 nm, the appearance of the peak attributed to toluene is reduced, and the overlap with the peak attributed to free sulfur is reduced.

Thus, when the measurement wavelength is 280 to 330 nm, the overlap of the peaks of toluene and free sulfur is reduced, enabling more accurate measurement.

The quantitative method described above can accurately measure the free sulfur content of a rubber composition, regardless of whether it is an unvulcanized rubber composition or a vulcanized rubber composition, in particular an unvulcanized rubber composition containing insoluble sulfur in which transition from insoluble sulfur to soluble sulfur may affect the measurement accuracy.

Moreover, the quantification of free sulfur according to the present invention can take measures against adhesion failure to adjacent components. Furthermore, there may be concerns that adhesion failure may occur when a large amount of sulfur required for adhesion between the cords and rubber blooms as free sulfur so that the amount of sulfur in the rubber decreases. However, measures against such adhesion failure can also be taken by adjusting the free sulfur content according to the present invention.

### EXAMPLES

The present invention is specifically described with reference to, but not limited to, examples.

The following describes the materials used below.
IR: polyisoprene rubber (IR2200) available from JSR Corporation
Insoluble sulfur: M95 available from Nippon Kanryu Industry Co., Ltd.
Soluble sulfur: powdered sulfur available from Tsurumi Chemical Industry Co., Ltd.
THF: tetrahydrofuran available from Wako Pure Chemical Industries, Ltd.
Toluene: toluene of special grade available from Kanto Chemical Co., Inc.

### <Preparation of sample>

According to the formulation shown in Table 1, IR and insoluble sulfur or soluble sulfur were roll-kneaded to obtain an unvulcanized rubber composition, which was then cut into pieces to prepare a sample (sample A or B) (size: 2 × 2 × 2 mm³ to 3 × 3 × 3 mm³) .

**[Table 1]**

| | Sample A | Sample B |
|---|---|---|
| IR | 100 | 100 |
| Soluble sulfur | 2 | |
| Insoluble sulfur | | 2 |

### <Comparative Example>

The sample A or B was immersed and left in THF for 24 hours to obtain an extract (temperature: 23°C, sample: 10 mg/mL).

The extract was separated by GPC using the apparatus and detector described below under the measurement conditions described below to obtain a chromatogram, and the amount of free sulfur was quantified based on the area of the peak attributed to free sulfur (measurement wavelength: 264 nm).

### <Example>

The sample A or B was immersed and left in toluene for 24 hours to obtain an extract (temperature: 23°C, sample: 10 mg/mL).

The extract was separated by GPC using the apparatus and detector described below under the measurement conditions described below to obtain a chromatogram, and the amount of free sulfur was quantified based on the area of the peak attributed to free sulfur (measurement wavelength: 300 nm).

Here, the quantification of free sulfur was performed using a calibration curve separately prepared using liquids (standard sample solutions) in which sulfur was dissolved in THF or toluene.
Apparatus: GPC available from Shimadzu Corporation
Detector: PDA detector available from Shimadzu Corporation
(Measurement conditions)
Column: SHODEX LF-804 × 2
Mobile phase: THF
Column temperature: 40°C
Flow rate: 1.0 mL/min
PDA detection wavelength: 264 nm, 300 nm

The free sulfur contents of the sample A (THF extraction) and the sample B (THF extraction) determined by the quantitative method of the comparative example and the free sulfur contents of the sample A (toluene extraction) and the sample B (toluene extraction) determined by the quantitative method of the example as described above are expressed as an index, with the free sulfur content of the extract of the sample A (THF extraction) determined by the quantitative method of the comparative example being taken as 100. FIG. 3 shows the indices. A higher index indicates a higher free sulfur content determined by the analysis.

When the quantitative method of the comparative example (THF extraction) was used to detect the free sulfur in the sample B containing insoluble sulfur, the insoluble sulfur was detected as free sulfur, and poor accuracy was obtained. In this case, transition from insoluble sulfur to soluble sulfur presumably occurred.

In contrast, when the quantitative method of the example (toluene extraction) was used to detect the free sulfur in the sample B containing insoluble sulfur, the insoluble sulfur was hardly detected as free sulfur, and accurate measurement of free sulfur was obtained. In this case, transition from insoluble sulfur to soluble sulfur was presumably sufficiently reduced.

## Claims

1. A method of quantification of free sulfur in a rubber composition, the method comprising
extracting the free sulfur in the rubber composition with toluene,
wherein the quantification of free sulfur is performed by chromatography,
**characterized in that** the free sulfur is detected using an ultraviolet-visible detector at a measurement wavelength of 280 to 330 nm.

## Patentansprüche

1. Verfahren zur Quantifizierung von freiem Schwefel in einer Kautschukzusammensetzung, wobei das Verfahren umfasst:
Extrahieren des freien Schwefels in der Kautschukzusammensetzung mit Toluol,
wobei die Quantifizierung von freiem Schwefel durch Chromatographie durchgeführt wird,
**dadurch gekennzeichnet, dass** der freie Schwefel unter Verwendung eines Ultraviolett-Sichtbar-Detektors bei einer Messwellenlänge von 280 bis 330 nm detektiert wird.

## Revendications

1. Procédé de quantification de soufre libre dans une composition de caoutchouc, le procédé comprenant
l'extraction du soufre libre dans la composition de caoutchouc avec du toluène,
dans lequel la quantification du soufre libre est effectuée par chromatographie,
**caractérisé en ce que** le soufre libre est détecté en utilisant un détecteur d'ultraviolet-visible à une longueur d'onde de mesure de 280 à 330 nm.
